# EUROPEAN PATENT APPLICATION

(11) **EP 4 095 256 A1**
(43) Date of publication of application: **30.11.2022**
(21) Application number: 20916071.2
(22) Date of filing: 23.01.2020
(51) Int. Cl.: C12Q 1/6806, C12Q 1/6869, C12N 15/10

(54) **DROPLET MICROFLUIDICS-BASED SINGLE CELL SEQUENCING AND APPLICATIONS**

(71) Applicant: DGI Tech (Qing Dao) Co., Limited, Qingdao, Shandong 266555 (CN)
(72) Inventor: LIU, Chuanyu, Qingdao, Shandong 266555 (CN); LIU, Longqi, Qingdao, Shandong 266555 (CN); HUANG, Yaling, Qingdao, Shandong 266555 (CN); YUAN, Yue, Qingdao, Shandong 266555 (CN); WANG, Mingyue, Qingdao, Shandong 266555 (CN); WANG, Zifei, Qingdao, Shandong 266555 (CN); WEI, Xiaoyu, Qingdao, Shandong 266555 (CN); LIU, Ya, Qingdao, Shandong 266555 (CN); WANG, Shanshan, Qingdao, Shandong 266555 (CN); WANG, Chunqing, Qingdao, Shandong 266555 (CN)
(74) Representative: Habermann, Hruschka & Schnabel
(86) International application number: PCT/CN2020/073968
(87) International publication number: WO 2021/147069

(57) **Abstract**

Provided in the present application are a sequencing library and applications thereof. The sequencing library provided comprises: a first nucleic acid molecule, the first nucleic acid molecule carrying a cell tag sequence and a droplet tag sequence; and a second nucleic acid molecule, the second nucleic acid molecule carrying an insert fragment and a cell tag sequence.

## Description

### FIELD

The present disclosure relates to the field of biotechnology, and particularly, to a droplet microfluidics-based single-cell sequencing and applications.

### BACKGROUND

Single-cell sequencing technology is one of the most popular technologies in the past decade, and has shown a wide range of application prospects in development, tumor-related scientific research, and disease diagnosis. At present, single-cell sequencing technology has been realized at different levels such as genome, transcriptome, epiome and proteome, and it has played an important role in the research of many scientific issues. However, the current single-cell sequencing technology faces many challenges in terms of data quality, throughput, cost, and operability, and there are still many technical barriers to real large-scale applications.

Omics sequencing technology based on a microfluidic system is one of the most concerned fields in recent years. Compared with traditional technologies, the micro-scale droplet field in microfluidics is characterized by its extremely small consumption of reagents, which can greatly reduce the consumption cost of expensive reagents. Each droplet with stable morphology can be regarded as an independent microreactor, which reduces cross-contamination and is easy to manipulate. The droplet's large specific surface area can also accelerate various reactions and heat transfer, which is ideal in the field of single-cell research. Compared with other microfluidic technologies, droplet microfluidics has gradually become a popular method for biochemical analysis. However, at present, most of the single-cell library construction and sequencing technologies based on droplet microfluidic platforms require an investment of 100,000 cells, but only a few thousand single cells can be recovered, resulting in a large amount of waste of cells. The invention and use of new biochemical strategies to reduce cell input and increase cell recovery is one of the most important research hotspots at present.

### SUMMARY

The present disclosure aims to solve at least one of the technical problems in the related art to a certain extent. When the single-cell sequencing is performed by using the droplet microfluidic technology, the input amount of cells is reduced, and the recovery rate and utilization rate of cells can be improved, to avoid the waste of cells, which is more suitable for the purpose of sequencing a small input amount of cells.

Single-cell sequencing based on droplet microfluidic technology has become an ideal method for single-cell research due to a microscale reagents and simple manipulation. Referring to the literature published on Cell Resource (Highly Parallel Genome-wide Expression Profiling of Individual Cells Using Nanoliter Droplets, Evan Z. Macosko et al., Cell 161, 1202-1214, May 21, 2015), the droplet microfluidic technology uses a water-in-oil droplet to encapsulate cells and microbeads, so as to obtain a droplet containing one cell and one microbead. The other components of the droplet include a lysate. The micro-particle contains a large number of identical barcodes (cell index sequences, i.e., cell barcodes, which are used as identifiers of reads from the same cell). During the continuous generation of droplets, the cell in the droplet is lysed by the lysate, releasing a large number of mRNAs, and at the same time, the mRNA can be captured by the microbead. After all the droplets are generated, the droplets are broken. The mRNA is reverse transcribed into cDNA, which is further amplified. Then, the cDNA is fragmented by a fragment enzyme and sequencing adapters are added to both ends thereof. Further, the cDNA with the adapters is sequenced.

However, the droplet microfluidic technology also has an obvious disadvantage, that is, the initial input number of cells is too high (usually more than 100,000), and the finally obtained cells only account for a very low proportion of the input cells (about 4%). One main reason is that the distribution of the two phases, cell and microbead, in the droplet is a random event, which conforms to the Poisson distribution. Single-cell sequencing is required to ensure that the droplet contains only one cell and one microbead, and there can be no more than 2 different cells or 2 different microbeads in one droplet. In order to meet this requirement, both the cells and microbeads need to be prepared at a very low concentration, so that a large number of droplets do not simultaneously encapsulate the cells or microbeads, or there is only one cell or only one microbead. In the end, a lot of cells were wasted, and the number of cells that meet the sequencing requirements only accounted for about 4% of the input cells, which results in a large amount of waste of cells. A focus of the present disclosure is about how to reduce the input number of cells and increase the proportion of obtained cells to avoid a large waste of cells, especially to be suitable for the single-cell sequencing of a small input amount of cells.

In the process of research, Applicant creatively thought about whether it is possible to encapsulate multiple microbeads in one droplet, and then recognize these microbeads of one droplet to increase the proportion of cells that meet the sequencing requirements. For example, a droplet index sequence can be added to each distinct droplet as a marker to identify reads from different droplets. A cell index sequence may also be present in the droplet as a marker to identify reads from different cells. The sequencing library constructed in this manner contains both the cell index sequence and the droplet index sequence. During sequencing, by identifying these index sequences, different reads can be attributed to different cells, thereby realizing single-cell sequencing. According to embodiments of the present disclosure, the microbeads in one droplet carries the cell index sequence on the surface thereof and contains capture sequences used for capturing insert fragments from cells (i.e., fragments whose sequencing information is to be obtained through sequencing), and nucleic acid molecules carrying the insert fragments and cell index sequences can be obtained in the finally formed sequencing library. At the same time, the insert fragments from the same cell may be captured by more than one microbeads carrying different cell index sequences, and they cannot be distinguished effectively after de-emulsification. Therefore, by adding the droplet index sequence to the droplet, since the droplet index sequence can be captured by the capture sequence on the surface of the microbead in the droplet at the same time, the nucleic acid molecules carrying both the cell index sequence and the droplet index sequence can be obtained in the finally formed sequencing library. Thereby, by sequencing the nucleic acid molecules carrying the insert fragments and the cell index sequences, and by sequencing the nucleic acid molecules carrying the cell index sequences and the droplet index sequences, the sequencing results are mutually verified to confirm the reads from the same cell, thereby obtaining the single-cell sequencing result.

Such an approach for constructing the sequencing library and performing the single-cell sequencing does not require that one droplet encapsulate only one microbead, and thus a very low concentration of microbeads is no longer needed. As a result, more cells can be captured by the microbeads, thereby reducing the input amount of cells. Specifically, the input amount of cells can be reduced to be less than 10,000 from more than 100,000, and the recovery rate of cells is also increased from 4% to more than 50%. A single experiment obtains 4,000 to 6,000 available cells, which can greatly reduce the cost of single-cell library construction and sequencing, and is conducive to various large-scale researches or tests carried out through single-cell sequencing. In addition, those skilled in the art understand that any other available vector having the same functions as the microbead may also fall within the protection scope of the present disclosure.

Specifically, the present disclosure provides the following technical solutions.

In a first aspect of the present disclosure, the present disclosure provides a sequencing library including: a first nucleic acid molecule carrying a cell index sequence and a droplet index sequence; and a second nucleic acid molecule carrying an insert fragment and the cell index sequence. As described above, the present disclosure confirms reads from the same cell by sequencing the nucleic acid molecule carrying the insert fragment and the cell index sequence, and sequencing the nucleic acid molecule carrying the cell index sequence and the droplet index sequence, according to the droplet index and cell index, to obtain the single-cell sequencing result.

According to embodiments of the present disclosure, the sequencing library described above may further include the following technical features.

According to an embodiment of the present disclosure, the second nucleic acid molecule further includes a Unique Molecular Identifier. The second nucleic acid molecule further carries the Unique Molecular Identifier, which can indicate reads from different genes to achieve accurate determination of different genes in cells.

According to an embodiment of the present disclosure, the Unique Molecular Identifier has a length ranging from 6nt to 15nt, preferably from 8nt to 12nt, and more preferably 10nt. The Unique Molecular Identifier having length from 6 to 15 bases may from 4⁶ to 4¹⁵ different sequences. For example, the length of Unique Molecular Identifiers may be 6nt, 7nt, 8nt, 9nt, 10nt, 11nt, 12nt, 13nt, 14nt or 15nt, etc., which can be used to indicate reads from different genes without occupying too much sequencing information or causing unnecessary waste.

According to an embodiment of the present disclosure, the insert fragment is formed based on an mRNA molecule or a DNA molecule. The insert fragment carried on the second nucleic acid molecule can be an mRNA molecule from a lysed cell, or a DNA molecule. As an example, the mRNA molecule usually carries a polyA sequence. In this way, when a microbead or any other vector is used for capture, a polyT sequence can serve as the corresponding capture sequence to realize the capture of the insert.

According to an embodiment of the present disclosure, the cell index sequence has a length ranging from 10nt to 16nt. The cell index sequence having a length of 10 to 16 bases may from 4¹⁰ to 4¹⁶ different sequences, which can be used to indicate different cells without occupying too much sequencing information or causing unnecessary waste.

According to an embodiment of the present disclosure, the droplet index sequence has a length ranging from 6nt to 15nt, preferably from 8nt to 12nt, and more preferably 10nt. The droplet index sequence having a length of 6 to 15 bases may from 4⁶ to 4¹⁵ different sequences. For example, the length of the droplet index sequences can be 6nt, 7nt, 8nt, 9nt, 10nt, lint, 12nt, 13nt, 14nt or 15nt, etc., which can be used to indicate sequencing reads from the one droplet without occupying too much sequencing information or causing unnecessary waste.

In a second aspect of the present disclosure, the present disclosure provides a droplet including a biological material containing a nucleic acid molecule; a droplet identification molecule carrying a droplet index sequence; and a first vector carrying a capture sequence and a cell index sequence. The capture sequence is configured to capture at least one of the nucleic acid molecule and the droplet identification molecule. According to an embodiment of the present disclosure, the first vector may further contain an amplification recognition sequence. As described above, the present disclosure provides a droplet containing both the droplet identification molecule and the vector, which can be used to indicate sequencing reads from the same cell. In addition, since one droplet contains only one cell, the amount of cells used can be reduced, thereby improving the utilization rate of cells during single-cell sequencing. Thus, the droplet is especially suitable for single-cell sequencing of a small input amount of cells.

According to an embodiment of the present disclosure, the above-mentioned droplet may further include the following technical features.

According to an embodiment of the present disclosure, the nucleic acid molecule is mRNA or DNA.

According to an embodiment of the present disclosure, the biological material is provided in a form of cell.

According to an embodiment of the present disclosure, each droplet includes one cell, and each droplet includes at least one vector.

According to an embodiment of the present disclosure, the droplet further contains a cell lysate. The biological material in the droplet, such as the cell, can be lysed by using the cell lysis reagent, thereby facilitating the capture of the nucleic acid molecule on the biological material by the capture sequence on the vector.

According to an embodiment of the present disclosure, the droplet identification molecule further includes the captured sequence, and the captured sequence is connected to the droplet index sequence. The droplet identification molecule further includes the captured sequence, and the captured sequence can be identified by the capture sequence on the vector, thereby facilitating the capture of the droplet identification molecule by the vector.

According to an embodiment of the present disclosure, the droplet identification molecule is in a form of a long-chain molecule, and the long-chain molecule includes many copies of the droplet identification molecules in tandem; and the many copies of droplet index sequences on the same long-chain molecule has an identical nucleic acid sequence. The droplet identification molecule is provided in the form of a long-chain molecule. The long-chain molecule contains multiple droplet identification molecules in tandem. By processing the long-chain molecule, for example, by inserting an endonuclease recognition sequence between the droplet identification molecules, followed by enzymatic digestion with endonucleases, the multiple droplet identification molecules can be obtained and used in different droplets to indicate sequencing reads from different droplets. The multiple droplet identification molecules can also be obtained in other ways. For example, some oligonucleotides carrying cell index sequences immobilized on beads as the first vector can be released, bonded to long-chain molecules, and then extended or amplified to obtain the droplet identification molecules.

According to an embodiment of the present disclosure, the long-chain molecule further includes an endonuclease recognition sequence disposed between two adjacent droplet identification sequences, and the droplet further includes an endonuclease configured to cleave the endonuclease recognition sequence. The droplet may further contain the endonuclease to identify the endonuclease recognition sequence, so as to easily process the long-chain molecule. In this way, a large number of droplet identification molecules can be quickly obtained and applied in different droplets.

According to an embodiment of the present disclosure, the endonuclease recognition sequence is a double-stranded sequence, and the droplet identification molecule is a single-stranded sequence.

According to an embodiment of the present disclosure, the droplet identification molecule is provided by a form of a second vector, the second vector carrying many copies of droplet identification molecules. According to an embodiment of the present disclosure, the plurality of droplet identification molecules is connected to the second vector through a covalent bond or any other connection manner. For example, it can be connected to the vector through a disulfide bond, which facilitates subsequent use of some covalent bond cleavage reagents, such as DTT, to break the covalent bond and release the droplet identification molecule, thereby facilitating the capture by the capture sequence carried on the first vector. Alternatively, the second vector can be a hydrogel microbead, in which the plurality of droplet identification molecules can be embedded, and the hydrogel can be melted under suitable reaction conditions to release the droplet identification molecules. The other connection manners mentioned can be those similar to covalent bonding, but do not break hydrogen bonds.

According to an embodiment of the present disclosure, the plurality of droplet identification molecules each further includes the captured sequence, and the captured sequence is connected to the droplet index sequence and reverse complementary with the capture sequence of the first vector. This facilitates the specific binding of the capture sequence carried on the first vector to the capture sequence, thereby capturing the droplet index sequence.

According to an embodiment of the present disclosure, the covalent bond is a disulfide bond.

According to an embodiment of the present disclosure, the droplet further includes: a cleavage reagent capable of cleaving connections between the plurality of droplet identification molecules and the second vector. The cleavage reagent mentioned herein can break the covalent bond or other modification connection between the above-mentioned droplet identification molecule and the second vector.

According to an embodiment of the present disclosure, the cleavage reagent is DTT.

According to an embodiment of the present disclosure, the droplet has a water-in-oil structure.

In a third aspect of the present disclosure, the present disclosure provides a suspension including a plurality of droplets described in any embodiment of the second aspect of the present disclosure, and the droplet index sequences of at least two droplets of the plurality of droplets have different nucleic acid sequences. By preparing the suspension containing a plurality of droplets, the suspension can be sequenced and analyzed for a desired sequencing result.

In a fourth aspect of the present disclosure, the present disclosure provides a cell dispersion including: a cell; and a long-chain molecule comprising a plurality of droplet index sequences in tandem. The plurality of droplet index sequences on a same long-chain molecule has an identical nucleic acid sequence. The provided cell dispersion can be combined with a vector, such as microbead, to prepare droplets by means of a microfluidic chip, followed by performing demulsification, library construction, sequencing, etc. on the droplets, so as to obtain the sequencing result of single cell in the cell dispersion.

According to an embodiment of the present disclosure, the cell dispersion described above may further include the following technical features.

According to an embodiment of the present disclosure, the cell is present in a form of single cell.

According to an embodiment of the present disclosure, the cell dispersion includes a plurality of long-chain molecules. The plurality of droplet index sequences on at least two long-chain molecules of the plurality of long chain molecules has different nucleic acid sequences.

According to an embodiment of the present disclosure, the long-chain molecule further includes an endonuclease recognition sequence located between two adjacent droplet index sequences of the plurality of droplet index sequences.

According to an embodiment of the present disclosure, the endonuclease recognition sequence is a double-stranded sequence, and each droplet index sequence is a single-stranded sequence.

According to an embodiment of the present disclosure, the long-chain molecule is in a form of DNA nanoball.

In a fifth aspect of the present disclosure, the present disclosure provides a cell dispersion including a cell; and a second vector carrying a plurality of droplet identification molecules. Each droplet identification molecule can be connected to the second vector by a covalent bond or any other connection manner, and each droplet identification molecule carries a droplet index sequence.

According to embodiments of the present disclosure, the cell dispersion provided in the fifth aspect may further include the following technical features.

According to an embodiment of the present disclosure, the cell is in a form of single cell.

According to an embodiment of the present disclosure, each droplet identification molecule further includes a captured sequence, and the captured sequence is connected to the droplet index sequence.

According to an embodiment of the present disclosure, the covalent bond is a disulfide bond.

In a sixth aspect of the present disclosure, the present disclosure provides a sequencing method. The sequencing method includes: sequencing the sequencing library described in any embodiment of the first aspect of the present disclosure to obtain a sequencing result composed of a plurality of sequencing reads; and classifying sources of the plurality of sequencing reads based on at least one of the cell index sequence, the droplet index sequence, or the Unique Molecular Identifier.

In a seventh aspect of the present disclosure, the present disclosure provides a cyclic nucleic acid molecule. The cyclic nucleic acid molecule includes a replication-initiating sequence, a captured sequence, and a droplet index sequence. The replication-initiating sequence, which is included in the cyclic nucleic acid molecule provided by the present disclosure, can be used for replication and amplification. In addition, the cyclic nucleic acid molecule provided by the present disclosure further contains the captured sequence to be identified by the capture sequence from the first vector, and the droplet index sequence to be used in droplets as markers indicating sequencing reads from one droplet.

According to an embodiment of the present disclosure, the cyclic nucleic acid molecule further includes an endonuclease recognition sequence. The endonuclease recognition sequence can be identified by the endonuclease, thereby facilitating the subsequent obtaining of a large number of identical chain nucleic acid molecules.

In an eighth aspect of the present disclosure, the present disclosure provides a method for preparing a long-chain molecule. The method includes: performing a rolling circle replication amplification on the cyclic nucleic acid molecule described in the sixth aspect of the present disclosure to obtain the long-chain molecule. Thus, long-chain molecules can be easily and quickly obtained.

In a ninth aspect of the present disclosure, the present disclosure provides a linear nucleic acid molecule. The linear nucleic acid molecule includes a 5'-end sequence and a 3'-end sequence, a replication-initiating sequence, a captured sequence, and a droplet index sequence. The 5'-end sequence and the 3'-end sequence constitute an endonuclease recognition sequence.

In a tenth aspect of the present disclosure, the present disclosure provides a method for analyzing single-cell nucleic acids. The method includes: providing a single-cell suspension containing dispersed single cells, and mixing the single-cell suspension with droplet identification molecules to obtain a cell suspension, each of the droplet identification molecules carrying a droplet index sequence; and placing the cell suspension, a first vector, and an oil at different positions of a microfluidic chip in such a manner that the cell suspension, the first vectors, and the oil pass through channel to obtain the droplet as defined in any embodiment of the second aspect of the present disclosure; performing demulsification and library construction on the droplet to obtain a sequencing library; and sequencing and analyzing the sequencing library to obtain nucleic acid information of the single cell.

According to an embodiment of the present disclosure, the above-described method for analyzing single-cell nucleic acids may further include the following technical features.

According to an embodiment of the present disclosure, a concentration of the single-cell suspension ranges from 100 to 200 cells per microliter, and wherein a concentration of the droplet identification molecules ranges from 10⁵ to 10⁸ copies per microliter.

According to an embodiment of the present disclosure, a concentration of the first vectors ranges from 2,000 to 3,000 per microliter.

Through the technical solutions provided by the present disclosure, the input amount of cells for droplet microfluidic single-cell library construction can be reduced to 10,000, and the cell recovery rate can reach 50% or greater. One single experiment obtains 4,000 to 6,000 available cells, which greatly reduces the cost of single-cell library construction and sequencing, and is conducive to the development of large-scale single-cell research projects.

Additional aspects and advantages of the present disclosure will be set forth, in part, from the following description, and will become apparent in part from the following description, or may be learned by practice of the present disclosure.

### BRIEF DESCRIPTION OF DRAWINGS

The above and/or additional aspects and advantages of the present disclosure will become apparent and readily understood from the following description of embodiments taken in conjunction with the accompanying drawings, in which:
FIG. 1 is a schematic diagram of a droplet according to an embodiment of the present disclosure;
FIG. 2 is a schematic diagram of a droplet according to an embodiment of the present disclosure;
FIG. 3 is a schematic diagram of a DNA oligo sequence according to an embodiment of the present disclosure;
FIG. 4 is a schematic diagram of a microfluidic chip according to an embodiment of the present disclosure;
FIG. 5 is a graph illustrating a quality inspection result of a cDNA product according to Example 1 of the present disclosure;
FIG. 6 is a graph illustrating a quality inspection result of a DNB (DNA nanoball) oligo amplification product containing droplet index sequences according to Example 1 of the present disclosure;
FIG. 7 is a graph illustrating a quality inspection result of a transcriptome library according to Example 1 of the present disclosure;
FIG. 8 is a graph illustrating a quality inspection result of a DNB oligo library containing droplet index sequences according to Example 1 of the present disclosure;
FIG. 9 illustrates graphs of results of analyzing and detecting the number of cells and the number of genes in a single cell according to a sequencing result of the transcriptome library according to Example 1 of the present disclosure;
FIG. 10 shows the number of cell barcodes corresponding to each DNB oligo according to Example 1 of the present disclosure;
FIG. 11 is a diagram illustrating a correlation analysis result according to Example 1 of the present disclosure;
FIG. 12 is a diagram illustrating a cell barcode pairing analysis result according to Example 1 of the present disclosure;
FIG. 13 is a graph illustrating a quality inspection result of a cDNA product according to Example 2 of the present disclosure;
FIG. 14 is a graph illustrating a quality inspection result of a DNB oligo library containing droplet index sequences according to Example 2 of the present disclosure;
FIG. 15 illustrates graphs of results of analyzing and detecting the number of cells and the number of genes in a single cell according to a sequencing result of a transcriptome library according to Example 2 of the present disclosure;
FIG. 16 illustrates a result of the molecules number of captured droplet index sequences corresponding to each combination of cell index sequence and droplet index sequence according to Example 2 of the present disclosure;
FIG. 17 shows the number of types of droplet index sequences corresponding to each cell index sequence according to Example 2 of the present disclosure;
FIG. 18 shows the number of cell index sequences corresponding to each droplet index sequence according to Example 2 of the present disclosure;
FIG. 19 is a diagram illustrating a correlation analysis result according to Example 2 of the present disclosure; and
FIG. 20 is a diagram illustrating a cell barcode paring analysis result according to Example 2 of the present disclosure.

### DESCRIPTION OF EMBODIMENTS

The embodiments of the present disclosure are described in detail below, and examples of the embodiments are illustrated in the accompanying drawings. The embodiments described below with reference to the accompanying drawings are illustrative and are intended to explain the present disclosure, and they should not be construed as limiting the present disclosure.

In the description of the present disclosure, the relevant terms herein are explained and illustrated, and these explanations and illustrations are only for the convenience of understanding the solutions, and should not be regarded as a limitation on the claimed solutions of the present disclosure.

Herein, a nucleic acid molecule may refer to an RNA molecule, a DNA molecule, or a modified DNA molecule and/or RNA molecule, or an unmodified DNA molecule and/or RNA molecule. Those skilled in the art can specifically determine, based on the context, whether it is an RNA molecule, or a DNA molecule, or both an RNA molecule and a DNA molecule. In addition, the connection of nucleic acid molecules, unless otherwise specified, refers to one of the most common connection through a 3'-5' phosphodiester bond. Unless otherwise specified, when these different nucleic acid molecules are connected, it is not required that the nucleic acid molecules are connected directly, and they may be connected through other nucleic acid molecules, as long as they are on the same nucleic acid chain or on the same ring.

Herein, "cell index sequence", "droplet index sequence" or "captured sequence", etc., is a fragment of nucleic acid molecule. As mentioned above, these nucleic acid molecules may be RNA or DNA molecules. The different expressions are only intended to indicate the different functions of these nucleic acid sequences. Those skilled in the art can understand the effects of the sequences represented by the various expressions according to the context.

For example, herein, the cell index sequence (cell barcode) can be used to indicate that the sequencing reads are from the same cell. The droplet index sequence (droplet barcode) can indicate that the sequencing reads are from one droplet. The capture sequence refers to a sequence used to pair with and thus capture other nucleic acid molecules. The captured sequence refers to a sequence capable recognizing the capture sequence, and usually, the captured sequence is reverse complementary with the capture sequence to capture nucleic acid molecules.

It is required to input a large input amount of cells when performing single-cell library construction and sequencing based on a droplet microfluidic platform. In order to solve such a problem, in the present disclosure, a plurality of microbeads can be encapsulated in one droplet during the preparation of droplets, and sequencing reads in these microbeads from one droplet are then determined to improve the utilization of cells.

According to an embodiment of the present disclosure, the droplet may contain a biological material, a droplet identification molecule, and a first vector. The biological material contains the nucleic acid molecules. The droplet identification molecule carries a droplet index sequence. The first vector carries a capture sequence and a cell index sequence. The capture sequence is configured to capture at least one of the nucleic acid molecule and the droplet identification molecule. The first vector may further carry an amplification recognition sequence.

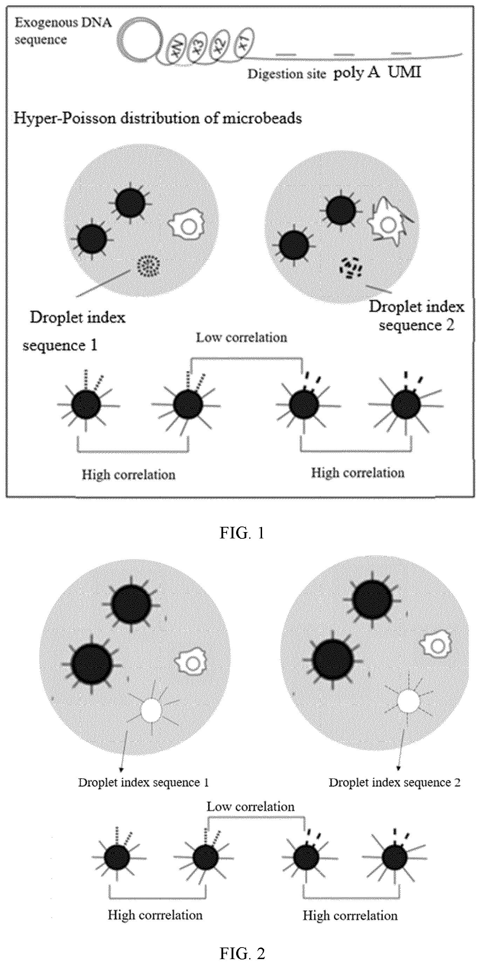

According to an embodiment of the present disclosure, the droplet identification molecule in the droplet can be obtained by means of DNB technology, as illustrated in FIG. 1. For example, a DNA fragment having a length of 100bp to 2,000bp and carrying a droplet index sequence, an endonuclease recognition sequence, a PCR adapter sequence, and polyA can be synthesized; an oligo-stranded nucleotide capable of anchoring to the DNA fragment can then be used as a primer for rolling circle linear amplification to obtain a DNA nanoball containing 20 to 200 copies of the DNA fragment.

The droplet identification molecule obtained by means of DNB technology is provided in the form of single strand, but the endonucleases recognize a double-stranded nucleic acid molecule. In this regard, the oligo-stranded nucleotide capable of anchoring to the endonuclease recognition sequence can then be hybridized with the above endonuclease recognition sequence, which is convenient for subsequent digestion with the endonuclease, thereby obtaining a large number of identical droplet identification molecules.

In addition, the DNA nanoball and the biological material can be pre-mixed, the vector and the endonuclease can be pre-mixed, and then droplet encapsulation can be performed using a conventional droplet microfluidic platform. The DNA nanoball is fragmented into small fragments under the action of restriction endonuclease. The small fragments are captured by the vectors (such as microbeads) with polyT, and the vectors from one droplet will capture the same droplet index sequences. As illustrated in FIG. 1, each droplet formed contain a different cell and DNA carrying a different droplet index sequence. After the subsequent reaction is completed, based on the similarity of the droplet index sequences on the microbeads, it can be determined which microbeads come from one droplet, i.e., the nucleic acid information corresponding to the same cell, can be identified.

According to an embodiment of the present disclosure, the concentration of DNA nanoballs is 10⁵ to 10⁸ copies per microliter in such a manner that a single droplet contains at least one DNA nanoball. Under this concentration condition, most of the single droplets contain one DNA nanoball; and a few of the single droplets contain two or more DNA nanoballs. In this case, multiple magnetic beads present in one droplet can capture two types of droplet index sequences, and multiple magnetic beads present in other droplets can capture other droplet index sequences. In combination with the correlation between encapsulation of multiple nanoballs and capture of multiple magnetic beads in the droplet, it is possible to calculate which magnetic beads are from one droplet.

According to an embodiment of the present disclosure, the droplet identification molecule in the droplet may be provided in a form of a vector, which is referred to as a second vector in order to be distinguished from the first vector carrying the cell index sequence. That is, the droplet identification molecule may be provided in the form of the second vector, and the second vector carries a plurality of droplet identification molecules. Each of the plurality of droplet identification molecules may be connected to the second vector by a covalent bond or other means. Each droplet identification molecule, in addition to carrying the droplet index sequence, may further contain the captured sequence, and the captured sequence is connected to the droplet index sequence. The droplet identification molecule can be connected to the vector through a covalent bond, for example, a disulfide bond. Thus, some covalent bond cleavage reagents, such as DTT, may be subsequently used to break the covalent bond and release the droplet identification molecule, thereby facilitating capture by the capture sequence carried on the first vector. Alternatively, the second vector can be a hydrogel microparticle, and the plurality of droplet identification molecules can be embedded in the hydrogel microparticle, and the hydrogel can be melted under suitable reaction conditions to release the droplet identification molecules.

According to an embodiment of the present disclosure, generally speaking, the applicant second vector may be a magnetic bead, and the size of the magnetic bead may generally be smaller than that of the first vector. The second vector carries a plurality of identical droplet index sequences. When droplets are formed subsequently, the covalent bond cleavage reagent in the droplet can destroy the covalent connections between the droplet index sequences and the second vector, without affecting the hydrogen bond connections between nucleic acid molecules, thereby releasing a large number of droplet index sequences in the droplet. The large number of droplet index sequences released can be captured by the first vector, and thus the vectors from one droplet will capture the same droplet index sequences. As illustrated in FIG. 2, each formed droplet contains a cell, and DNA carrying unique droplet index sequences. After the subsequent reaction is completed, based on the similarity of the droplet index sequences on the microbeads, the microbeads from the same droplet, that is, the nucleic acid information corresponding to the same cell, can be identified.

Then, the conventional droplet microfluidic technology is used to perform demulsification, followed by reverse transcription reaction, enzyme digestion of empty oligonucleotides on the surface of the vector, cDNA amplification, library construction, sequencing and analysis, thereby completing single-cell sequencing.

The solutions of the present disclosure will be explained below in conjunction with examples. Those skilled in the art can understand that the following examples are only used to illustrate the present disclosure, but they should not be construed as limiting the scope of the present disclosure. The specific technique or condition indicated in the examples shall be that described in the literature in the related art or in the specification of used product, unless specifically indicated. The reagents or instruments used without indication of the manufacturers are the conventional products that can be purchased.

### Example 1

Example 1 provides a method for preparing a sequencing library and performing high-throughput sequencing. The provided method is implemented through several steps, including: DNA nanoball preparation, single cell suspension preparation, microbead preparation, droplet generation, demulsification, reverse transcription RT reaction, enzyme digestion, cDNA amplification, fragmentase library construction, high-throughput sequencing, etc.

### 1. Preparation of DNA Nanoballs

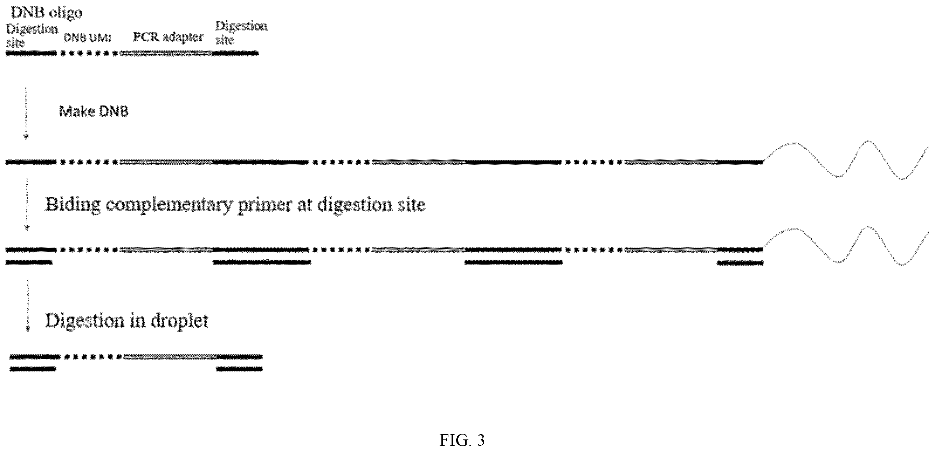

Referring to FIG. 3, the DNB oligo sequence was first synthesized and then cyclized to prepare a DNB. Then, by bonding to a primer complementary to the digestion site, the endonuclease recognition sequence on the DNB is annealed into a double-stranded sequence, which is suitable for the subsequent digestion with an endonuclease.

### 1.1 Primer sequence synthesis

Artificial synthesis of the following sequence:

### DNB oligo sequence:

5’-Pho-

CTAATAATACAATATTTTTTTTTTTTTTTTTTTTVAACATGANNNNNNNNNNGCTA

AAGTCGGAGGCCAAGCGGTCTTAGGAAGACAAAGATAGGTCT-3’

Explanation: UMI digestion site separated and located at two ends of the sequence (which will be connected after cyclization) + a number of protecting bases for restriction digestion at the two ends of the digestion site + PolyT sequence (forming PolyA sequence after forming the DNB) + fixed sequence + DNB UMI + PCR adapter sequence (because it cannot be interrupted for library construction, the adapter can be identified and amplified during Sample index amplification).

Specifically, "**CTAATA**" and "**GGTCT**" shown in bold with single underline in the above sequence represent the digestion site of Bsal restriction endonuclease, which is separated and located the two ends of the sequence (to be connected with each other after cyclization). "ATACAATA" and "AGATA" denoted with the dotted line in the above sequence are protecting bases for restriction digestion, and for improving the efficiency of restriction digestion. In the above sequence, "TTTTTTTTTTTTTTTTTTTTTV" is synthesized into the PolyA sequence after making DNB, and the PolyA sequence can be complementary to and thus be captured by the oligonucleotide sequence on the magnetic bead. The 10nt bases "N" in the sequence represent a random sequence for labeling the droplet (i.e., the droplet index sequence as mentioned above), and each DNB carries a different index sequence. The "AAGTCGGAGGCCAAGCGGTCTTAGGAAGACAA" denoted with double-underline in the above sequence is a PCR adapter, which is complementary to the library adapter PCR primer for amplification during library construction.

### Splint oligo:

5'-GTATTATTAGAGACCTATCT-3'. The Splint oligo is a cyclization complementary primer.

Digestion site complementary sequence (Digestion site complementary oligo): 5'-**AGATAGGTCTCTAATAATACA**-3'

Explanation: 5'-end and 3'-end of the synthesized DNB-oligo sequence contain the digestion site sequences to be cyclized and spliced together to form a complete digestion site, thereby forming a DNB-oligo ring. Then, the formed DNB-oligo ring is used for rolling circle replication and amplification to form DNBs, the synthesized single strand is a complementary strand of the DNB-oligo ring, and then the complementary sequence of the digestion site is added. Therefore, the complementary sequence is consistent with the sequences at the two ends of the original oligo.

### 1.2 Single-stranded DNB Oligo cyclization

a. 200 to 400 ng of DNB oligo sequences was placed into a new 0.2 ml PCR tube and the tube was added with TE Buffer (Cat. No. AM9849) to a total volume of 47µl.
b. 3.0µl of 20 µM Splint oligo was added, mixed and centrifuged briefly to precipitate to the bottom of the tube.
c. The tube was placed in a PCR amplifier and incubated at 95°C for 3 minutes.
d. Immediately after the reaction, the PCR tube was transferred to ice and stood for 5 minutes.
e. A single-strand cyclization reaction solution was prepared on ice: 3.2µl of TE Buffer, 6µl of 10x TA buffer (Cat. No. Y038), 0.6µl of 100 mM ATP (Cat. No. R1441), and 0.2µl of 600 U/µl T4 DNA Ligase (Cat. No.: BGE004).
f. 10µl of the prepared single-strand cyclization reaction solution was pipetted into the above PCR tube, mixed by vortex, and centrifuged briefly to precipitate the reaction solution to the bottom of the tube.
g. The PCR tube was placed on the PCR amplifier, incubated at 37°C for 30 minutes, and thermally covered at 75°C.
h. A digestion reaction solution was prepared on ice: 1.0µl of TE Buffer, 0.4µl of 10x TA buffer, 1.95µl of 20 U/µl EXO I (Cat. No. 01E010MS), and 0.65µl of 100 U/µl EXO III (Cat. No. 01E011HS).
i. 4µl of the digestion reaction solution was pipetted into the single-stand cyclization product, mixed by vortex, and centrifuged briefly to precipitate the reaction solution to the bottom of the tube.
j. The tube was placed in the PCR amplifier, incubated at 37°C for 30 minutes, and thermally covered at 75°C. The single-stranded oligonucleotide strands, which were not cyclized, were digested and degraded by the digestion reaction solution.
k. After completion of the reaction, 3µl of 0.5M EDTA (Cat. No.: AM9260) was added, mixed well and centrifuged.
l. 90µl of PEG32 (Cat. No.: Y041) magnetic beads was used to recover the above digestion products, 32µl of TE Buffer was used to elute the DNAs, and the concentration of single-stranded product was detected with Qubit^{®} ssDNA Assay Kit (Cat. No.: Q10212).

### 1.3 RCA and digestion site hybridization reaction

a. The following reaction system was prepared with make DNB kit (Cat. No. 1000012552) on ice: 4µl of single-stranded product ssDNA (10 ng), 4µl of 10X Phi29 Buffer, 1.0µl of 20µM Splint oligo, 16µl of TE Buffer, and 15µl of H₂O.
b. 40µl of the above reaction mixture was evenly mixed with a vortex shaker and centrifuged in a mini centrifuge for 5s.
c. The tube was placed in the PCR amplifier, incubated 95°C for 1 min, 65°C for 1 min, and 40°C for 1 min, the thermal cover at 105°C.
d. DNB polymerase mixture solution II (LC) was taken, centrifuged briefly for 5s, and stored in an ice box for later use.
e. After completion of the reaction in step c, the PCR tube was taken out and added with 40µl of make DNB enzyme Mix and 4µl of make DNB enzyme Mix II to the tubes while being placed on ice.
f. The mixture was evenly mixed with a vortex shaker, and centrifuged for 5s in a mini centrifuge.
g. The tube was placed in the PCR amplifier, incubated at 30°C for 20 minutes, the task was terminated immediately when the temperature dropped to 4°C; the PCR tube was transferred to the ice box, added with 20µl of DNB stop buffer, and slowly mixed 5-8 times with a flaring pipette, without shaking or centrifuging.
h. The DNB concentration was measured using the Qubit^{®} ssDNA Assay Kit.
i. 20µl of the above reaction product, DNBs, was added into a new PCR tube, and 10µl of 10µM Digestion site complementary oligo, 10µl of 10x Cutsmart buffer (Cat. No.: B7204S), and 60µl of H₂O were added and gently pipetted 5-8 times, without shaking and centrifuging.
j. The tube was placed in the PCR amplifier, incubated at 55°C for 2 minutes, 40°C for 2 minutes, 30°C for 2 minutes, 20°C for 2 minutes, 10°C for 2 minutes, and 4°C for 2 minutes.
   Explanation: this process is the annealing binding of primers complementary to the digestion sites. Generally, the complementary primers on the DNB are at 55°C during sequencing by the sequencer, and thus 55°C gradient annealing strategy was adopted.
k. 10µl of the above product was added into a new PCR tube, and 90µl of EB buffer was added for quantitative dilution, and the mixture was placed on ice for later use.

### 2. Preparation of single cell suspension

2.1 Single cell suspensions for 293T cell line and NIH3T3 cell line were prepared by trypsin digestion, washed with PBS (Cat. No.: 10010031) (containing 0.04% BSA) 1-2 times, and filtrated with 40µm cell sieve.

2.2 The cell concentration was detected with a cell counting plate or counter.

2.3 Based on the cell concentration, 20,000 cells were taken through pipette each time, and the cell pellet was collected after centrifugation at 300-500g, and 100µl of Cell Resuspension Buffer was added to resuspend the cells.

2.4 Before loading the sample, 10µl of the DNB product obtained in step k was added in the above step 1.3 to the Cell Resuspension Buffer, mixed evenly with a flaring pipette tip, and placed on ice for later sample loading.

### 3. Preparation of microbeads

### 3. Microbead preparation

3.1 With reference to the article published by BGI about stLFR magnetic bead preparation method (Efficient and unique co-barcoding of second-generation sequencing reads from long DNA molecules enabling cost effective and accurate sequencing, haplotyping, and de novo assembly, https://genome.cshlp.org/content/early/2019/04/02/gr.245126.118), magnetic beads (purchased from Spherotech, USA, Cat. No.: SVM-200-4, https://www.spherotech.com/coa_mag_par.htm) were subjected to surface oligonucleotide coating to obtain magnetic beads with oligonucleotides on the surface. 200µl of the magnetic beads (220,000) were pipetted and added into 0.2ml PCR tube, placed on a magnetic stand for 2 min, and the supernatant was removed.

3.2 The PCR tube was removed from the magnetic stand, and added with 200µl of 1x Buffer D (1 mM EDTA, 9 mg/ml 85% KOH) to suspend the magnetic beads.

3.3 The tube was incubated at room temperature for 5 min.

3.4 The tube was placed on the magnetic stand for 2 min, and the supernatant was removed.

3.5 The PCR tube was kept on the magnetic stand, added with 200µl of 1x Buffer D, stood for 30 s, and the supernatant was removed.

3.6 200µl of LSWB was added and stood for 30s, and then the supernatant was removed.

3.7 The previous step repeated.

3.8 200µl of Lysis Buffer (6% Ficoll PM-400 (Cat. No.: 17-0300-10), 0.2% Sarkosyl (Cat. No.: L7414), 20mM EDTA, 200mM Tris pH 7.5 (Cat. No.: T2944-1L), H₂O) was added and stood for 30s, and then the supernatant was removed. The PCR tube was removed from the magnetic stand, added with 100µl of Lysis Buffer and 5.5µl of 1M DTT, and finally added with 4.5µl of BsaI (Cat. No.: R0535S) restriction endonuclease. The magnetic beads were suspended with a low adsorption pipette tip on ice.

### 4. Droplet generation

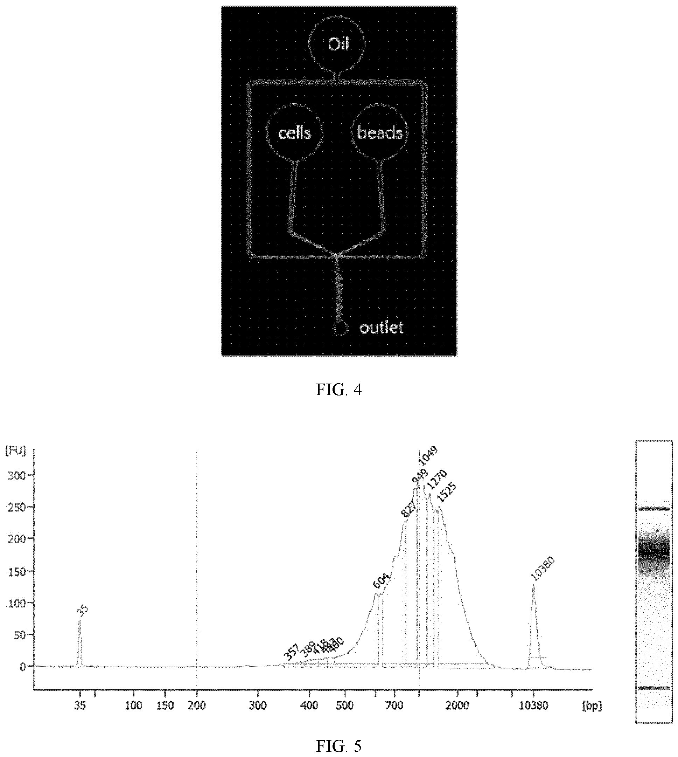

4.1 A protective film on a surface of a chip was teared off, and the chip was placed in a chip slot region of a droplet generator. The chip has a structure as illustrated in FIG. 4.

4.2 An A-end of a connection tube on a collection cap (contacting the connection tube at the bottom of the collection tube) was placed into an outlet hole of the chip.

4.3 A 50ml syringe was placed on a fixing holder and the push rod was pushed to an initial position. A blunt-end syringe needle was used to connect the syringe with the B-end of the connecting tube on the cap of the collection tube (without contacting the connecting tube at the bottom of the collection tube).

4.4 200µl of droplet generation oil was added to the collection tube, the collection cap was tightened, and the collection tube was placed upright on the fixing holder.

4.5 The cells were evenly mixed through gentle pipetting, and 100µl of the single cell suspension (obtained in step 2 above) was added to the cell well of the chip, under the premise that the pipette tip touched the bottom of the well.

4.6 The magnetic beads were evenly mixed through gentle pipetting, and 100µl of magnetic beads (obtained in step 3 above) were added to the bead well of the chip, under the premise that the pipette tip touched the bottom of the well.

4.7 350µl of the droplet generation oil was immediately added to the oil well of the chip.

4.8 The push rod of the syringe was quickly pulled to the slot position, and the push rod was clamped at the slot.

4.9 A timer was activated for 8 min and the droplets were collected.

4.10 After 8 minutes, the collection cap on the collection tube was immediately unscrewed. The connection tube of the outlet hole of the chip was pulled out, and the connection tube was stretched vertically. The droplets in the tube flow into the collection tube, and then an ordinary collection tube cap was used for replacement.

4.11 The collection tube stood still at room temperature for 20 min to fully bind mRNA molecules to the magnetic beads.

### 5. Demulsification

5.1 In order to prepare demulsification reagent, 10ml of 6X SSC (Cat. No. 15557-036) and 200µl of PFO (Cat. No. 370533-25G) were added to a 15ml centrifuge tube.

5.2 The filter device and the vacuum pump were connected, the pressure parameter was adjusted to 0.01MPa or 100mbar, and the vacuum pump was turned on.

5.3 20ml of 6X SSC was added to pretreat the device.

5.4 When no liquid remained on the filter membrane, all the liquids in the collection tube were evenly poured on the surface of the filter membrane, the collection tube was washed twice with 2ml of 6X SSC, and the cleaning solutions were together poured into the filter device.

5.5 10ml of demulsification reagent was vigorously inverted and mixed and then poured into the filter device quickly.

5.6 When no liquid remained on the filter membrane, 30ml of 6X SSC was continuously added to wash the magnetic beads.

5.7 When no liquid remained on the filter membrane, the vacuum pump was turned off and the vacuum pump from the filter device were disconnected.

5.8 The filter port of the filter device was closed with a syringe or rubber stopper.

5.9 1.0ml of collection buffer was added with a pipette, and the entire surface of the filter membrane was subjected to about 20 times of gentle pipetting to suspend the magnetic beads.

5.10 The collection solution containing the magnetic beads was transferred to a 1.5ml low adsorption centrifuge tube.

5.11 1.0ml of collection buffer was added with a pipette, and the entire surface of the filter membrane was subjected about 10 times of gentle pipetting to suspend the remaining magnetic beads.

5.12 The collection solution containing magnetic beads was transferred to a 1.5ml low-adsorption centrifuge tube, and the tube was placed on a magnetic stand and stood still for 2 min, and the supernatant was slowly removed.

5.13 The centrifuge tube was removed from the magnetic stand. 100µl of collection buffer was used to suspend the magnetic beads adsorbed on one side of the two centrifuge tubes in turn, and the liquid was transferred to 0.2ml low adsorption PCR tube.

5.14 100µl of collection buffer was used to suspend the magnetic beads adsorbed on one side of the two centrifuge tubes again, and the liquid was transferred to the above-mentioned 0.2 ml low adsorption PCR tube.

5.15 The PCR tube with magnetic beads was placed on the magnetic stand and stood still for 2 min, and then the supernatant was removed.

5.16 The magnetic beads were kept in the adsorbed state, 200µl of 6X SSC was added and stood still for 30s, and then the supernatant was removed.

5.17 200µl of 5X FS Buffer was added and stood still for 30s, and the supernatant was slowly removed to avoid attracting magnetic beads.

### 6. Reverse transcription reaction

6.1 Reverse transcription reaction system was prepared on ice: 5µl of H₂O, 20µl of 5x First-Strand Buffer (Cat. No.: 01E022MS), 20µl of 5M Betaine (Cat. No.: B0300-1VL), 10µl of 10mM dNTPs (Cat. No.: N0447L), 7.5µl of 100mM MgCl₂ (Cat. No. 20-303), 5µl of 50µM Template switch oligo, 5µl of 100mM DTT (Cat. No. 01E022MS), 5µl of 200U/µl Alpha reverse transcriptase (Cat. No. 01E022MS), and 2.5µl of 40U/µl RNase inhibitor (Cat. No. 01E019MS).

The above-mentioned Alpha reverse transcriptase is an engineered MMLV reverse transcriptase, which can recognize ssDNA as a template for complementary synthesis.

6.2 100µl of the reverse transcription reaction system was pipetted and added to the PCR tube containing magnetic beads, and mixed by repeatedly pipetting.

6.3 Reverse transcription reaction was performed according to the following conditions: 42°C, 90 min; and 10 cycles (50°C, 2 min; 42°C, 2 min), with thermal cover at 75°C. Due to the sedimentation of the magnetic beads, the magnetic beads were evenly mixed through gentle pipetting every 20 min, and the reaction continued after a brief centrifugation.

6.4 After completion of the reaction, the tube was centrifuged briefly, placed on the magnetic stand, stood still for 2 min, and the reaction solution was removed.

6.5 The PCR tube was removed from the magnetic stand, 200µl of TE-SDS was added and shaken to evenly mix, and the reaction was terminated.

6.6 After a brief centrifugation, the tube was placed on the magnetic stand, stood still for 2 min, and then the liquid was removed.

6.7 The magnetic beads were kept in the adsorbed state, 200µl of TE-TW was added, and the mixture stood still for 30s, and then the supernatant was removed.

6.8 The previous step repeated.

6.9 The magnetic beads were kept in the adsorbed state, 200µl of 10mM Tris (pH8.0) was added, and the mixture stood still for 30s, and then the supernatant was removed.

### 7. Digestion of empty oligos failing to capture mRNA molecules on surfaces of microbeads

7.1 Digestion reaction system: 170µl of H₂O, 20µl of 10x EXO I Buffer, and 10µl of EXO I enzyme.

7.2 200µl of the digestion reaction system was pipetted and added to the PCR tube containing magnetic beads, and evenly mixed by vortex.

7.3 After brief centrifugation, the tube was placed in a PCR amplifier, and incubated at 37°C for 45 min, with thermal cover at 75°C. The magnetic beads were evenly mixed through gentle pipetting every 15 min, and the reaction continued after brief centrifugation.

7.4 After completion of the reaction, the tube was centrifuged briefly, placed on the magnetic stand, stood still for 2 min, and the reaction solution was removed.

7.5 The PCR tube was removed from the magnetic stand, 200µl of TE-SDS was added and shaken to evenly mix, and the reaction was terminated

7.6 After brief centrifugation, the tube was placed on a magnetic stand, stood still for 2 min, and then the liquid was removed.

7.7 The magnetic beads were kept in the adsorbed state, 200µl of TE-TW was added, and the mixture stood still for 30s, and then the supernatant was removed.

7.8 The PCR tube was removed from the magnetic stand, 200µl of TE-TW was added to suspend the magnetic beads.

7.9 After brief centrifugation, the tube was placed on the magnetic stand, stood still for 2 min, and then the liquid was removed.

7.10 The magnetic beads were kept in the adsorbed state, 200µl of H₂O was added, and the mixture stood still for 30s, and then the supernatant was removed.

### 8. cDNA amplification

8.1 A PCR reaction system was prepared: 41µl of H₂O, 8µl of 10µM Tn Primer, 2µl of 1µM reverse primer, 50µl of 2x KAPA HiFi Hotstart Ready mix (Cat. No.: KK2602).

8.2 PCR reaction was performed according to the following conditions: 95°C, 3 min; 13 to 20 cycles (98°C, 20s; 58°C, 20s; 72°C, 3 min).

8.3 After the completion of PCR, the PCR product was purified and recovered by using 60µl of (0.6X) AMPure XP Beads (Cat. No.: A63881) (pre-equilibrated at room temperature for 30 minutes), the oligo small fragment product was recovered in the supernatant by using 200µl of (2X) AMPure XP beads, and the concentration was detected by using Qubit dsDNA HS Kit (Cat. No. Q32854).

8.4 A DNB-Oligo purified product secondary amplification PCR reaction system was prepared: 11µl of H₂O, 2µl of 10µM V4-Phos-Tn-C Primer, 2µl of 10µM V2-N7-index-n Primer, 25µl of 2x KAPA HiFi Hotstart Ready mix, and 10µl of DNB-Oligo purified product obtained in step 8.3.

8.5 PCR reaction was performed according to the following conditions: 95°C, 3 min; 6 to 10 cycles (98°C, 20s; 58°C, 20s; 72°C, 15s).

8.6 The oligo secondary amplification product was recovered by using 200µl of (2X) AMPure XP beads, and the concentration was detected using the Qubit dsDNA HS Kit.

### 9. Fragmentase library construction

9.1 The fragmentase was taken and evenly mixed by shaking for 5s, and the tube was centrifuged briefly and placed on ice for later use.

9.2 50 to 300ng of cDNAs to be interrupted was added into a new 0.2ml PCR tube, a volume thereof ≤16µl, and added with H₂O to 16µl.

9.3 4.0µl of the prepared fragmentation reaction solution (2µl fragmentase (Cat. No.: M0348L) and 2µl of 10x fragmentase buffer (Cat. No.: B0349) were pipetted and added into the cDNA tube, and mixed by vortex for 3 times, 3s each time, and centrifuged briefly to collect the reaction solution to the bottom of the tube.

9.4 The PCR tube was placed on the PCR amplifier and incubated at 37°C for 10 min.

9.5 30µl of 0.1M EDTA was added to the PCR tube, evenly mixed by vortex, and the reaction was terminated.

9.6 The reaction solution was collected to the bottom of the tube through a brief centrifugation, and placed on ice.

9.7 Fragment selection was performed on the fragmentation product by using 0.6x + 0.4x (i.e., 30µl + 20µl) AMPure XP beads, and the DNAs were eluted with 42µl of H₂O.

9.8 End repair reaction solution was prepared on ice: 2.3µl of H₂O, 5µl of 10x PNK buffer (Cat. No. B9040L), 1.2µl of 5:1 dATP: dNTP mix, 0.6µl of 10U/µl T4 Polynucleotide Kinase (Cat. No. Y9040L), 0.6µl of 3U/µl T4 DNA polymerase (Cat. No. P7080L), 0.2µl of 5U/µl rTaq (Cat. No. R500Z), and 0.1µl of 5U/µl Klenow fragment (Cat. No. P7060L).

9.9 10µl of the end repair reaction solution was pipetted and added into the selected fragmentation product, evenly mixed by vortex, and centrifuged briefly to collect the reaction solution to the bottom of the tube.

9.10 The PCR tube was incubated on the PCR amplifier at 37°C for 30 min, and at 65°C for 15min.

9.11 After completion of the reaction, the reaction solution was placed on ice.

9.12 An adapter ligation reaction solution was prepared on ice: 3.6µl of H₂O, 3µl of 10x PNK buffer, 5µl of 10µM Adapters mix, 0.8µl of 100mM ATP (Cat. No. R1441), 16µl of 50% PEG 8000 (Cat. No. EB-0.5P8K-250), and 1.6µl of 600U/µl T4 DNA Ligase (Cat. No. BGE004).

9.13 30µl of the prepared adapter ligation reaction solution was slowly pipetted and added to the end repair product, evenly mixed by vortex, and centrifuged briefly to collect the reaction solution to the bottom of the tube.

9.14 The PCR tube was placed on the PCR amplifier and incubated at 23°C for 60 min.

9.15 After completion of the reaction, 20µl of TE Buffer was added to make the total sample volume to reach 100µl.

9.16 The ligation product was purified and recovered by using 50µl of AMPure XP Beads, and the ligation product was eluted by using 48µl of H₂O.

9.17 2µl of 10µM PCR Primer and 50µl of 2x KAPA HiFi Hotstart Ready mix were added and evenly mixed by shaking.

9.18 PCR reaction was performed according to the following conditions: 95°C, 3min; 11 cycles (98°C, 20s; 58°C, 20s; 72°C, 30s).

9.19 After completion of PCR, the PCR product was screened by using 0.6x + 0.2x (i.e., 60µl + 20µl) AMPure XP Beads, DNAs were eluted using 42µl of TE Buffer, and the concentration was detected using Qubit dsDNA HS Kit.

### 10. High-throughput sequencing

10.1 A total of 200 to 400ng of the library was added into a new 0.2ml PCR tube, where a pooling ratio of the PCR product of cDNAs of the same sample after fragmentation and the oligo secondary PCR product was 9:1 (or, separate cyclization and then pooling). TE Buffer was added to reach a total volume of 47µl.

10.2 3.0µl of 20µM Splint Oligo primer was added and evenly mixed, and centrifuged briefly to precipitate to the bottom of the tube.

10.3 The tube was placed in a PCR amplifier and incubated at 95°C for 3 min.

10.4 Immediately after completion of the reaction, the PCR tube was transferred onto ice and stood still for 5 min.

10.5 A single strand cyclization reaction solution was prepared on ice: 3.2µl of TE Buffer, 6µl of 10x TA buffer, 0.6µl of 100mM ATP, and 0.2µl of 600U/µl T4 DNA Ligase.

10.6 10µl of the prepared single strand cyclization reaction solution was pipetted and added into the above PCR tube, evenly mixed by vortex, and centrifuged briefly to collect the reaction solution to the bottom of the tube.

10.7 The PCR tube was placed on the PCR amplifier and incubated at 37°C for 30 min.

10.8 The digestion reaction solution was prepared on ice: 1.0µl of TE Buffer, 0.4µl of 10x TA buffer, 1.95µl of 20U/µl EXO I, and 0.65µl of 100U/µl EXO III.

10.9 4µl of the digestion reaction solution was pipetted and added to the single strand cyclization product, evenly mixed by vortex, and centrifuged briefly to collect the reaction solution to the bottom of the tube.

10.10 The tube was placed on the PCR amplifier and incubated at 37°C for 30 min.

10.11 After completion of the reaction, 3µl of 0.5M EDTA was added and mixed, and the mixture was centrifuged.

10.12 The above digestion product was recovered by using 90µl of PEG32 magnetic beads, DNAs were eluted with 32µl of TE Buffer, and the concentration of single-stranded cyclic library was detected by using the Qubit^{®} ssDNA Assay Kit.

10.13 The qualified library was sequenced. The sequencing parameters: 41+100+10.

The experimental product and library quality inspection result are as follows:

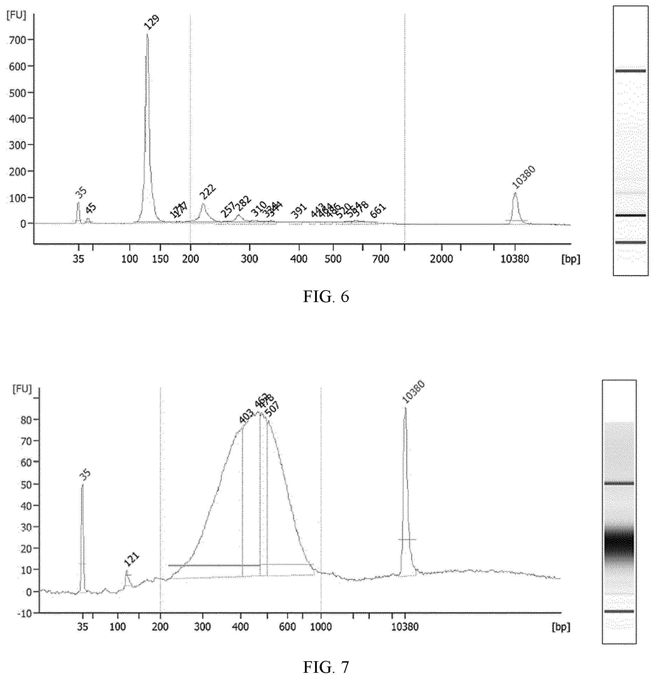

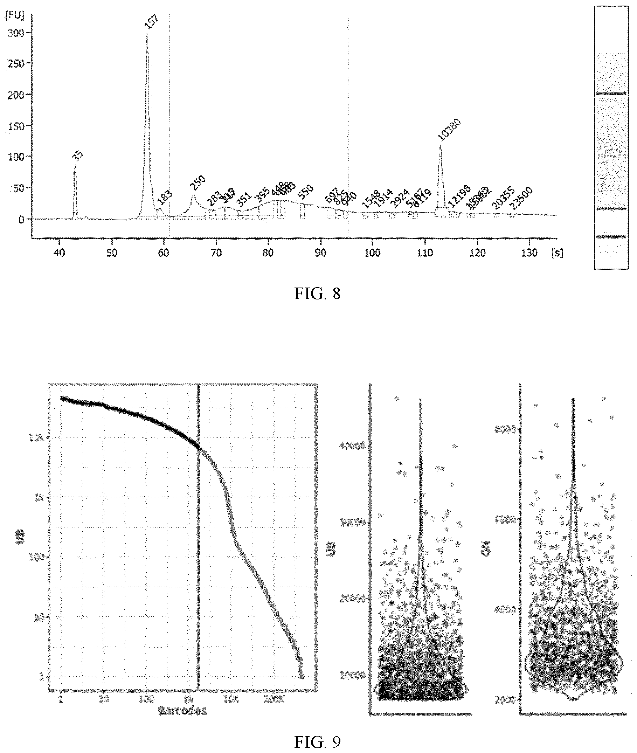

FIG. 5 is a graph illustrating the quality inspection result of the obtained cDNA product. FIG. 6 is a graph illustrating the quality inspection result of the obtained DNB oligo amplification product containing droplet index sequences. FIG. 7 is a graph illustrating the quality inspection result of the obtained transcriptome library. FIG. 8 is a graph illustrating the quality inspection result of the obtained DNB oligo library containing droplet index sequences.

After sequencing analysis, the results are shown in Table 1 below and FIG. 9:

**Table 1 Transcriptome sequencing results**

| | |
|---|---|
| Estimated number of cells | 1689 |
| Reads in cell | 0.421 |
| Mean UMI counts per cell | 11710 |
| Mean genes per cell | 3441 |

Table 1 and FIG. 9 show the results of detecting the number of cells and the number of genes in a single cell according to the analysis of the transcriptome sequencing result. In FIG. 9, UB represents the number of UMI counts, and GN represents the number of genes.

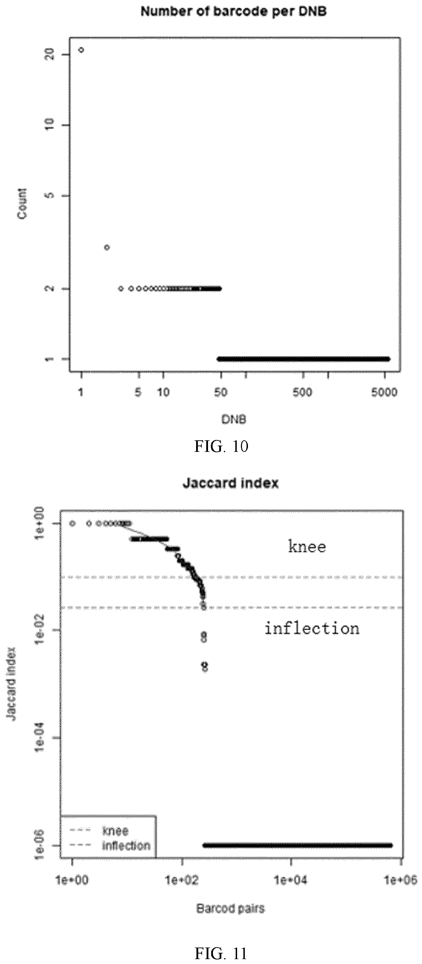

FIG. 10 shows the number of cell barcodes corresponding to each DNB oligo. It can be seen from this figure that most of the DNB-oligos each correspond to one cell barcode, and a small part of the DNB-oligos each corresponds to 2 or more cell barcodes. For the case that each DNB-oligo corresponds to 2 or more barcodes, Jaccard index can be used to analyze the correlation.

FIG. 11 shows the correlation analysis result. Jaccard index, also known as Jaccard similarity coefficient, is used to compare sets of limited samples in terms of their similarity and difference. The greater the Jaccard coefficient, the higher the sample similarity. The correlation between a series of barcode pairs (cell barcode pairs) can be obtained from the analysis, and it can be seen that some beads are highly correlated, illustrating potential droplets containing multiple cells. The correlation of beads of most droplets containing a single cell was at the lowest level.

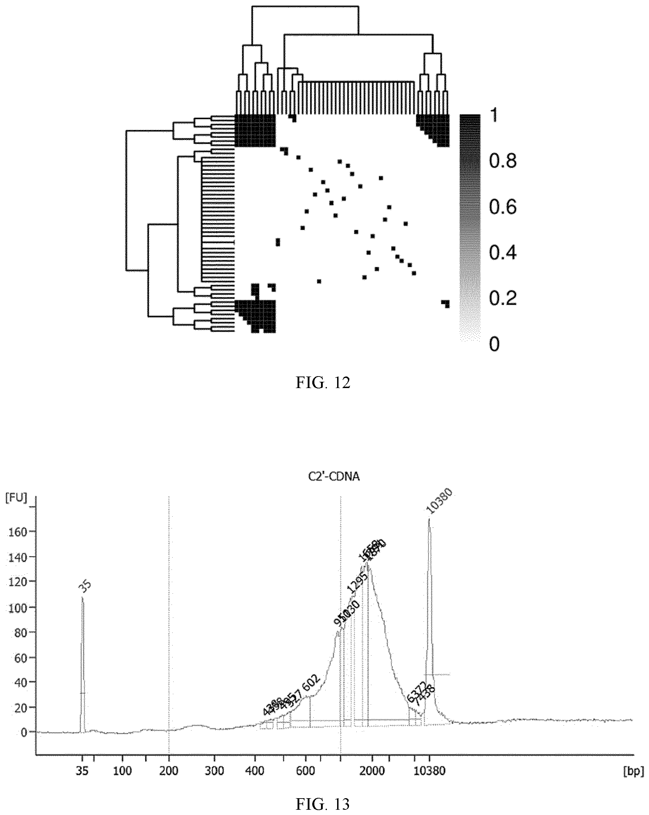

FIG. 12 shows the results of cell barcode pairing analysis. The rows and columns in FIG. 12 are all barcodes, and the darker the color, the higher the correlation. The rows or columns that are connected by line segments indicate that they potentially originate from the same droplet.

### Example 2

Example 2 provides a method for preparing a sequencing library and performing high-throughput sequencing. The provided method is implemented through several steps, including: barcode vector microbead preparation, single cell suspension preparation, microbead preparation, droplet generation, demulsification, reverse transcription RT reaction, enzyme digestion, cDNA amplification, fragmentase library construction, high-throughput sequencing, etc.

### 1. Preparation of microbead vectors carrying droplet index sequences

1.1 Coupling incubation of microbead vector oligonucleotides carrying droplet index sequences:
For each group, 200pmol of modified droplet index sequence oligonucleotides were taken and incubated with 1mg of Dynabeads^{™} M-280 Streptavidin magnetic beads at room temperature for 2 hours. After incubation, the magnetic beads were attracted to the magnetic stand and washed 3 times, and the magnetic beads were stored in low salt buffer. Buffer for incubation and washing of magnetic beads: (B&W) Buffer (1X) (5mM Tris-HCl (pH 7.5), 0.5mM EDTA, 1M NaCl).

The used droplet index sequence oligonucleotides:

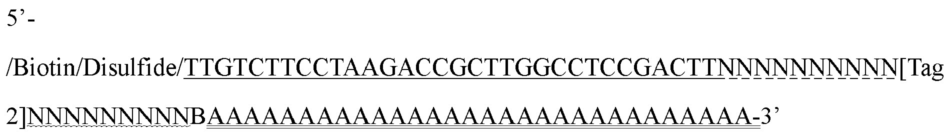

The sequence denoted with single line represents a PCR adapter sequence, and the following 10 bases represent the droplet index sequence. A total of 12 types were used in this example, and the following sequence denoted with a wavy line represents random bases for optimizing a sequencing quality of the barcode region. The base B immediately following the random bases is a degenerate base, which may be G, C or T. The sequence denoted with double lines represents a polyA sequence, which is used to be captured by an oligonucleotide on another magnetic bead (i.e., the magnetic bead carrying the cell index sequence) in the droplet. The 5'-end of this oligonucleotide strand has Biotin medication and disulfide bond modification.

### 1.2 Mixing and counting of droplet barcode vector microbeads

After 12 groups of barcode vector microbeads were prepared through incubation according to the above step, each group was mixed with an equal amount of magnetic beads, and the mixed barcode vector microbeads were counted for the last time to obtain the microbead concentration.

### 2. Preparation of single cell suspension

2.1 Single cell suspension for 293T cell line was prepared by trypsin digestion, washed with PBS (Cat. No.: 10010031) (containing 0.04% BSA) 1-2 times, and filtrated with 40µm cell sieve.

2.2 The cell concentration was detected with a cell counting plate or counter.

2.3 Based on the cell concentration, 20,000 cells were taken through pipette each time and the cell pellet was collected after centrifugation at 300-500g, and 100µl of Cell Resuspension Buffer was added to resuspend the cells.

2.4 2.2 million mixed barcode vector microbeads obtained in the above step 1.2 were added to the Cell Resuspension Buffer before sample loading. The specific volume was determined according to the concentration. The mixture was placed on ice for sample loading.

### 3. Microbead preparation

3.1 With reference to the literature published by BGI about a stLFR magnetic bead preparation method (Efficient and unique co-barcoding of second-generation sequencing reads from long DNA molecules enabling cost effective and accurate sequencing, haplotyping, and de novo assembly, https://genome.cshlp.org/content/early/2019/04/02/gr.245126.118), magnetic beads (purchased from Spherotech, USA, Cat. No.: SVM-200-4, https://www.spherotech.com/coa_mag_par.htm) were subjected to surface oligonucleotide coating to obtain magnetic beads with oligonucleotides on the surfaces thereof. 200µl of magnetic beads (220,000) were pipetted and added to into 0.2ml PCR tube, placed on a magnetic stand for 2 min, and then the supernatant was removed.

3.2 The PCR tube was removed from the magnetic stand, and added with 200µl of 1x Buffer D (1 mM EDTA, 9mg/ml 85% KOH) to suspend the magnetic beads.

3.3 The tube was incubated at room temperature for 5 min.

3.4 The tube was placed on the magnetic stand for 2 min, and then the supernatant was removed.

3.5 The PCR tube was kept on the magnetic stand, added with 200µl of 1x Buffer D, and stood still for 30s to remove the supernatant.

3.6 200µl of LSWB was added and stood for 30s, and then the supernatant was removed.

3.7 The previous step repeated.

3.8 200µl of Lysis Buffer (6% Ficoll PM-400 (Cat. No.: 17-0300-10), 0.2% Sarkosyl (Cat. No.: L7414), 20mM EDTA, 200mM Tris pH 7.5 (Cat. No.: T2944-1L), H₂O) was added and stood for 30s, and then the supernatant was removed. The PCR tube was removed from the magnetic stand, added with 99µl of Lysis Buffer and 11µl of 1M DTT to obtain a final concentration of 100mM DTT in this buffer. The magnetic beads were suspended with a low adsorption pipette tip on ice.

### 4. Droplet generation

4.1 A protective film on a surface of a chip was teared off, and the chip was placed in a chip slot region of a droplet generator. The chip has a structure as illustrated in FIG. 4.

4.2 An A-end of a connection tube on a collection cap (contacting the connection tube at the bottom of the collection tube) was placed into an outlet hole of the chip.

4.3 A 50ml syringe was placed on a fixing holder and the push rod was pushed to an initial position. A blunt-end syringe needle was used to connect the syringe with the B-end of the connecting tube on the cap of the collection tube (without contacting the connecting tube at the bottom of the collection tube).

4.4 200µl of droplet generation oil was added to the collection tube, the collection cap was tightened, and the collection tube was placed upright on the fixing holder.

4.5 The cells were evenly mixed through gentle pipetting, and 100µl of the single cell suspension (obtained in step 2 above) was added to the cell well of the chip, under the premise that the pipette tip touched the bottom of the well.

4.6 The magnetic beads were evenly mixed through gentle pipetting, and 100µl of magnetic beads (obtained in step 3 above) were added to the bead well of the chip, under the premise that the pipette tip touched the bottom of the well.

4.7 350µl of the droplet generation oil was immediately added to the oil well of the chip.

4.8 The push rod of the syringe was quickly pulled to the slot position, and the push rod was clamped at the slot.

4.9 A timer was activated for 8 min and the droplets were collected.

4.10 After 8 minutes, the collection cap on the collection tube was immediately unscrewed. The connection tube of the outlet hole of the chip was pulled out, and the connection tube was stretched vertically. The droplets in the tube flow into the collection tube, and then an ordinary collection tube cap was used for replacement.

4.11 The collection tube stood still at room temperature for 20 min to fully bind mRNA molecules to the magnetic beads.

### 5. Demulsification

5.1 In order to prepare demulsification reagent, 10ml of 6X SSC (Cat. No. 15557-036) and 200µl of PFO (Cat. No. 370533-25G) were added to a 15ml centrifuge tube.

5.2 The filter device and the vacuum pump were connected, the pressure parameter was adjusted to 0.01Mpa or 100mbar, and the vacuum pump was turned on.

5.3 20ml of 6X SSC was added to pretreat the device.

5.4 When no liquid remained on the filter membrane, all the liquids in the collection tube were evenly poured on the surface of the filter membrane, the collection tube was washed twice with 2ml of 6X SSC, and the cleaning solutions were together poured into the filter device.

5.5 10ml of demulsification reagent was vigorously inverted and mixed and then poured into the filter device quickly.

5.6 When no liquid remained on the filter membrane, 30ml of 6X SSC was continuously added to wash the magnetic beads.

5.7 When no liquid remained on the filter membrane, the vacuum pump was turned off and the vacuum pump from the filter device were disconnected.

5.8 The filter port of the filter device was closed with a syringe or rubber stopper.

5.9 1.0ml of collection buffer was added with a pipette, and the entire surface of the filter membrane was subjected to about 20 times of gentle pipetting to suspend the magnetic beads.

5.10 The collection solution containing the magnetic beads was transferred to a 1.5ml low adsorption centrifuge tube.

5.11 1.0ml of collection buffer was added with a pipette, and the entire surface of the filter membrane was subjected about 10 times of gentle pipetting to suspend the remaining magnetic beads.

5.12 The collection solution containing magnetic beads was transferred to a 1.5ml low-adsorption centrifuge tube, and the tube was placed on a magnetic stand and stood still for 2 min, and the supernatant was slowly removed.

5.13 The centrifuge tube was removed from the magnetic stand. 100µl of collection buffer was used to suspend the magnetic beads adsorbed on one side of the two centrifuge tubes in turn, and the liquid was transferred to 0.2ml low adsorption PCR tube.

5.14 100µl of collection buffer was used to suspend the magnetic beads adsorbed on one side of the two centrifuge tubes again, and the liquid was transferred to the above-mentioned 0.2 ml low adsorption PCR tube.

5.15 The PCR tube with magnetic beads was placed on the magnetic stand and stood still for 2 min, and then the supernatant was removed.

5.16 The magnetic beads were kept in the adsorbed state, 200µl of 6X SSC was added and stood still for 30s, and then the supernatant was removed.

5.17 200µl of 5X FS Buffer was added and stood still for 30s, and the supernatant was slowly removed to avoid attracting magnetic beads.

### 6. Reverse transcription reaction

6.1 Reverse transcription reaction system was prepared on ice: 5µl of H₂O, 20µl of 5x First-Strand Buffer (Cat. No.: 01E022MS), 20µl of 5M Betaine (Cat. No.: B0300-1VL), 10µl of 10mM dNTPs (Cat. No.: N0447L), 7.5µl of 100mM MgCl₂ (Cat. No. 20-303), 5µl of 50µM Template switch oligo, 5µl of 100mM DTT (Cat. No. 01E022MS), 5µl of 200U/µl Alpha reverse transcriptase (Cat. No. 01E022MS), and 2.5µl of 40U/µl RNase inhibitor (Cat. No. 01E019MS).

The above-mentioned Alpha reverse transcriptase is an engineered MMLV reverse transcriptase, which can recognize ssDNA as a template for complementary synthesis.

6.2 100µl of the reverse transcription reaction system was pipetted and added to the PCR tube containing magnetic beads, and mixed by repeatedly pipetting.

6.3 Reverse transcription reaction was performed according to the following conditions: 42°C, 90 min; and 10 cycles (50°C, 2 min; 42°C, 2 min), with thermal cover at 75°C. Due to the sedimentation of the magnetic beads, the magnetic beads were evenly mixed through gentle pipetting every 20 min, and the reaction continued after a brief centrifugation.

6.4 After completion of the reaction, the tube was centrifuged briefly, placed on the magnetic stand, stood still for 2 min, and the reaction solution was removed.

6.5 The PCR tube was removed from the magnetic stand, 200µl of TE-SDS was added and shaken to evenly mix, and the reaction was terminated.

6.6 After a brief centrifugation, the tube was placed on the magnetic stand, stood still for 2 min, and then the liquid was removed.

6.7 The magnetic beads were kept in the adsorbed state, 200µl of TE-TW was added, and the mixture stood still for 30s, and then the supernatant was removed.

6.8 The previous step repeated.

6.9 The magnetic beads were kept in the adsorbed state, 200µl of 10mM Tris (pH8.0) was added, and the mixture stood still for 30s, and then the supernatant was removed.

### 7. Digestion of empty oligos failing to capture mRNA molecules on surfaces of microbeads

7.1 Digestion reaction system: 170µl of H₂O, 20µl of 10x EXO I Buffer, and 10µl of EXO I enzyme.

7.2 200µl of the digestion reaction system was pipetted and added to the PCR tube containing magnetic beads, and evenly mixed by vortex.

7.3 After brief centrifugation, the tube was placed in a PCR amplifier, and incubated at 37°C for 45min, with thermal cover at 75°C. The magnetic beads were evenly mixed through gentle pipetting every 15 min, and the reaction continued after brief centrifugation.

7.4 After completion of the reaction, the tube was centrifuged briefly, placed on the magnetic stand, stood still for 2 min, and the reaction solution was removed.

7.5 The PCR tube was removed from the magnetic stand, 200µl of TE-SDS was added and shaken to evenly mix, and the reaction was terminated

7.6 After brief centrifugation, the tube was placed on a magnetic stand, stood still for 2min, and then the liquid was removed.

7.7 The magnetic beads were kept in the adsorbed state, 200µl of TE-TW was added, and the mixture stood still for 30s, and then the supernatant was removed.

7.8 The PCR tube was removed from the magnetic stand, 200µl of TE-TW was added to suspend the magnetic beads.

7.9 After brief centrifugation, the tube was placed on the magnetic stand, stood still for 2 min, and then the liquid was removed.

7.10 The magnetic beads were kept in the adsorbed state, 200µl of H₂O was added, and the mixture stood still for 30s, and then the supernatant was removed.

### 8. cDNA amplification

8.1 A PCR reaction system was prepared: 46µl of H₂O, 4µl of 10µM Tn Primer, and 50µl of 2x KAPA HiFi Hotstart Ready mix (Cat. No.: KK2602).

8.2 PCR reaction was performed according to the following conditions: 95°C, 3 min; 13 to 20 cycles (98°C, 20s; 58°C, 20s; 72°C, 3 min).

8.3 After the completion of PCR, the PCR product was purified and recovered by using 60µl of (0.6X) AMPure XP Beads (Cat. No.: A63881) (pre-equilibrated at room temperature for 30 minutes), the oligo small fragment product was recovered in the supernatant by using 200µl of (2X) AMPure XP beads, and the concentration was detected by using Qubit dsDNA HS Kit (Cat. No. Q32854).

8.4 A droplet molecular barcode purification product secondary amplification PCR reaction system was prepared: 11µl of H₂O, 2µl of 10µM V4-Phos-Tn-C Primer, 2µl of 10µM V2-N7-index-n Primer, 25µl of 2x KAPA HiFi Hotstart Ready mix, and 10µl of oligo small fragment purification product of step 8.3.

8.5 PCR reaction was performed according to the following conditions: 95°C, 3min; 6 to 10 cycles (98°C, 20s; 58°C, 20s; 72°C, 15s).

8.6 The oligo secondary amplification product was recovered by using 200µl of (2X) AMPure XP Microbeads, and the concentration was detected using the Qubit dsDNA HS Kit.

### 9. Fragmentase library construction

9.1 The cDNA product was subjected to the following library construction procedures: taking out the fragmentase, shaking and mixing for 5s, centrifuging briefly, and placing on ice for later use.

9.2 50 to 300ng of cDNAs to be interrupted was added into a new 0.2ml PCR tube, a volume thereof ≤16µl, and added with H₂O to 16µl.

9.3 4.0µl of the prepared fragmentation reaction solution (2µl fragmentase (Cat. No.: M0348L) and 2µl of 10x fragmentase buffer (Cat. No.: B0349) were pipetted and added into the cDNA tube, and mixed by vortex for 3 times, 3s each time, and centrifuged briefly to collect the reaction solution to the bottom of the tube.

9.4 The PCR tube was placed on the PCR amplifier and incubated at 37°C for 10 min.

9.5 30µl of 0.1M EDTA was added to the PCR tube, evenly mixed by vortex, and the reaction was terminated.

9.6 The reaction solution was collected to the bottom of the tube through a brief centrifugation, and placed on ice.

9.7 Fragment selection was performed on the fragmentation product by using 0.6x + 0.4x (i.e., 30µl + 20µl) AMPure XP beads, and the DNAs were eluted with 42µl of H₂O.

9.8 End repair reaction solution was prepared on ice: 2.3µl of H₂O, 5µl of 10x PNK buffer (Cat. No. B9040L), 1.2µl of 5:1 dATP: dNTP mix, 0.6µl of 10U/µl T4 Polynucleotide Kinase (Cat. No. Y9040L), 0.6µl of 3U/µl T4 DNA polymerase (Cat. No. P7080L), 0.2µl of 5U/µl rTaq (Cat. No. R500Z), and 0.1µl of 5U/µl Klenow fragment (Cat. No. P7060L).

9.9 10µl of the end repair reaction solution was pipetted and added to the selected fragmentation product, evenly mixed by vortex, and centrifuged briefly to collect the reaction solution to the bottom of the tube.

9.10 The PCR tube was incubated on the PCR amplifier at 37°C for 30 min, and at 65°C for 15min.

9.11 After completion of the reaction, the reaction solution was placed on ice.

9.12 An adapter ligation reaction solution was prepared on ice: 3.6µl of H2O, 3µl of 10x PNK buffer, 5µl of 10µM Adapters mix, 0.8µl of 100mM ATP (Cat. No. R1441), 16µl of 50% PEG 8000 (Cat. No. EB-0.5P8K-250), and 1.6µl of 600U/µl T4 DNA Ligase (Cat. No. BGE004).

9.13 30µl of the prepared adapter ligation reaction solution was slowly pipetted and added to the end repair product, evenly mixed by vortex, and centrifuged briefly to collect the reaction solution to the bottom of the tube.

9.14 The PCR tube was placed on the PCR amplifier and incubated at 23°C for 60 min.

9.15 After completion of the reaction, 20µl of TE Buffer was added to make the total sample volume reach 100µl.

9.16 The ligation product was purified and recovered by using 50µl of AMPure XP Beads, and the ligation product was eluted by using 48µl of H₂O.

9.17 2µl of 10µM PCR Primer and 50µl of 2x KAPA HiFi Hotstart Ready mix were added and evenly mixed by shaking.

9.18 PCR reaction was performed according to the following conditions: 95°C, 3min; 11 cycles (98°C, 20s; 58°C, 20s; 72°C, 30s).

9.19 After completion of PCR, the PCR product was screened by using 0.6x + 0.2x (i.e., 60µl + 20µl) AMPure XP Beads, DNAs were eluted using 42µl of TE Buffer, and the concentration was detected using Qubit dsDNA HS Kit.

### 10. High-throughput sequencing

10.1 A total of 200 to 400ng of the library was added into a new 0.2ml PCR tube, where a pooling ratio of the PCR product of cDNAs of the same sample after fragmentation and the oligo secondary PCR product was 9:1 (or, separate cyclization and then pooling). TE Buffer was added to reach a total volume of 47µl.

10.2 3.0µl of 20µM Splint Oligo primer was added and evenly mixed, and centrifuged briefly to precipitate to the bottom of the tube.

10.3 The tube was placed in a PCR amplifier and incubated at 95°C for 3 min.

10.4 Immediately after completion of the reaction, the PCR tube was transferred onto ice and stood still for 5min.

10.5 A single strand cyclization reaction solution was prepared on ice: 3.2µl of TE Buffer, 6µl of 10x TA buffer, 0.6µl of 100mM ATP, and 0.2µl of 600U/µl T4 DNA Ligase.

10.6 10µl of the prepared single strand cyclization reaction solution was pipetted and added into the above PCR tube, evenly mixed by vortex, and centrifuged briefly to collect the reaction solution to the bottom of the tube.

10.7 The PCR tube was placed on the PCR amplifier and incubated at 37°C for 30 min.

10.8 The digestion reaction solution was prepared on ice: 1.0µl of TE Buffer, 0.4µl of 10x TA buffer, 1.95µl of 20U/µl EXO I, and 0.65µl of 100U/µl EXO III.

10.9 4µl of the digestion reaction solution was pipetted and added to the single strand cyclization product, evenly mixed by vortex, and centrifuged briefly to collect the reaction solution to the bottom of the tube.

10.10 The tube was placed on the PCR amplifier and incubated at 37°C for 30 min.

10.11 After completion of the reaction, 3µl of 0.5M EDTA was added and mixed, and the mixture was centrifuged.

10.12 The above digestion product was recovered by using 90µl of PEG32 magnetic beads, DNAs were eluted with 32µl of TE Buffer, and the concentration of single-stranded cyclic library was detected by using the Qubit^{®} ssDNA Assay Kit.

10.13 The qualified library was sequenced. The sequencing parameters: 41+100+10.

The experimental product and library quality inspection result are as follows:

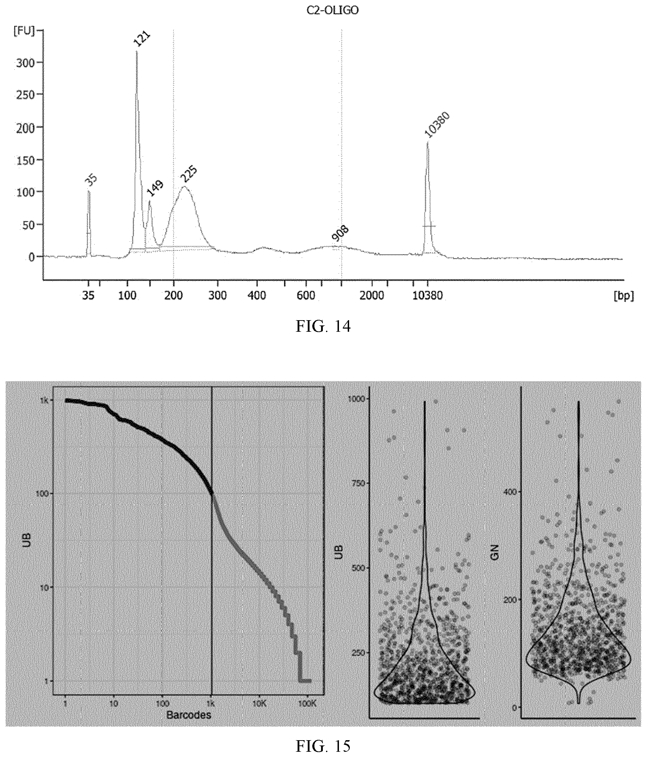

FIG. 13 is a graph illustrating the quality inspection result of the obtained cDNA product. FIG. 14 is a graph illustrating the quality inspection result of the obtained oligo amplification product containing droplet index sequences.

After sequencing analysis, the results are shown in Table 2 below and FIG. 15:

**Table 2 Transcriptome sequencing results.**

| | |
|---|---|
| Estimated number of cells | 1055 |
| Reads in cell | 0.276 |
| Mean UMI counts per cell | 218 |
| Mean genes per cell | 140 |

Table 2 and FIG. 15 show the results of detecting the number of cells and the number of genes in a single cell according to the analysis of the transcriptome sequencing result. In FIG. 15, UB represents the number of UMI counts, and GN represents the number of genes.

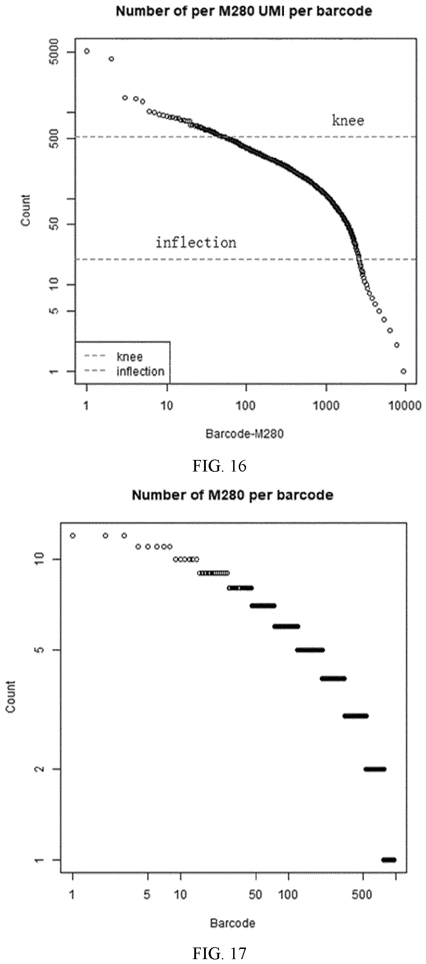

FIG. 16 shows the result of the number of molecules of captured droplet index sequences corresponding to each combination of cell index sequence and droplet index sequence, i.e., the number of pieces of droplet index sequences of one type captured by each cell index sequence. The shown result indicates that most of cell index sequences each captured several hundred droplet index sequences, indicating that the droplet index sequences can be efficiently dissociated from the magnetic beads and captured and labeled by the magnetic beads carrying the cell index sequence in effective quantity, which is sufficient to support subsequent analysis.

FIG. 17 shows the number of types of droplet index sequences corresponding to each cell index sequence. Each cell index sequence corresponds to 2 to 8 types of droplet index sequences. When the types of droplet index sequences are various enough, the cell index sequences can be grouped based on the combination of types of droplet index sequences captured by the magnetic beads carrying the cell index sequences. The magnetic beads carrying the cell index sequences and coming from the same droplet can be determined by correlation calculation.

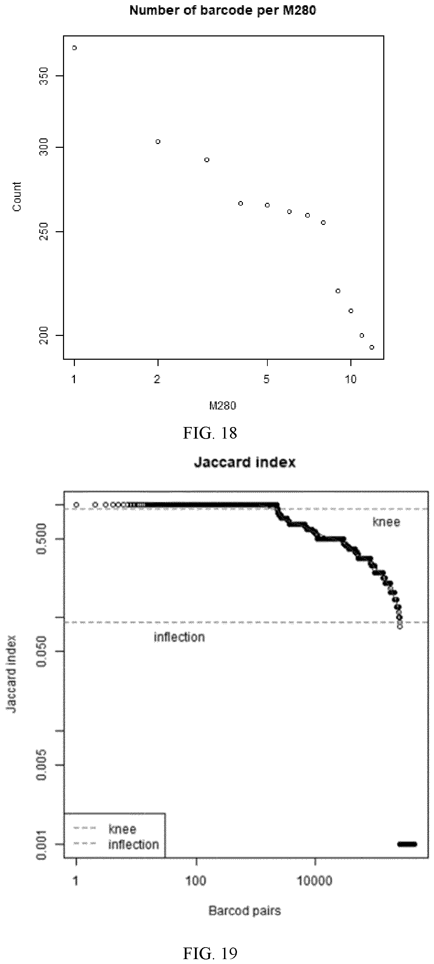

FIG. 18 shows the number of types of cell index sequences corresponding to each droplet index sequence. In view of this figure, the number of cell barcode types corresponding to each droplet index sequence can be known. In the present embodiment, there are 12 types of droplet index sequences and more than 1,000 types of cell index sequences, and thus, each type of droplet index sequence corresponds to many types of cell index sequences. By increasing the types of droplet index sequences, each type of droplet index sequence can correspond to fewer types of cell index sequences, so as to increase the degree of distinction of cell index sequences.

FIG. 19 shows the correlation analysis result. Jaccard index, also known as the Jaccard similarity coefficient, is used to compare sets of limited samples in terms of their similarity and difference. The greater the Jaccard coefficient, the higher the sample similarity. According to the analysis, the correlation between a series of barcode pairs can be obtained, and it can be seen that the correlation of some cell index sequences calculated based on the droplet index sequence is relatively high.

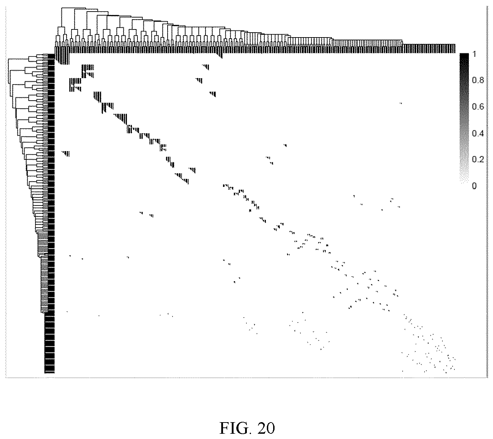

FIG. 20 illustrates the results of cell barcode pairing analysis, in which the rows and columns represent barcodes. The darker the color, the higher the correlation. The rows or columns that are connected by line segments indicate that they potentially originate from the same droplet.

In the specification, description with reference to the terms "an embodiment," "some embodiments," "example", "specific example" or "some examples", etc., mean that specific features, structures, materials, or characteristics described in connection with the embodiment or example is included in at least one embodiment or example of the present disclosure. In this specification, schematic representations of the above terms are not necessarily directed to the same embodiment or example. Furthermore, the particular features, structures, materials or characteristics described may be combined in any suitable manner in any one or more embodiments or examples. Furthermore, those skilled in the art may combine the different embodiments or examples described in this specification, as well as the features of the different embodiments or examples, as long as they do not conflict each other.

Although the embodiments of the present disclosure have been illustrated and described above, it should be understood that the above-mentioned embodiments are exemplary and should not be construed as limiting the present disclosure. Those skilled in the art may make changes, modifications, replacements and variations to the above embodiments within the scope of the present disclosure.

## Claims

1. A sequencing library, comprising:
a first nucleic acid molecule carrying a cell index sequence and a droplet index sequence; and
a second nucleic acid molecule carrying an insert fragment and the cell index sequence.

2. The sequencing library according to claim 1, wherein the second nucleic acid molecule further comprises a Unique Molecular Identifier.

3. The sequencing library according to claim 1, wherein the Unique Molecular Identifier has a length ranging from 6nt to 15nt.

4. The sequencing library according to claim 1, wherein the Unique Molecular Identifier has a length ranging from 8nt to 12nt.

5. The sequencing library according to claim 1, wherein the insert fragment is formed based on an mRNA molecule or a DNA molecule.

6. The sequencing library according to claim 1, wherein the cell index sequence has a length ranging from 10nt to 16nt.

7. The sequencing library according to claim 1, wherein the droplet index sequence has a length ranging from 6nt to 15nt.

8. The sequencing library according to claim 1, wherein the droplet index sequence has a length ranging from 8nt to 12nt.

9. A droplet, comprising:
a biological material containing a nucleic acid molecule;
a droplet identification molecule carrying a droplet index sequence; and
a first vector carrying a capture sequence and a cell index sequence,
wherein the capture sequence is configured to capture at least one of the nucleic acid molecule and the droplet identification molecule.

10. The droplet according to claim 9, wherein the nucleic acid molecule is mRNA or DNA.

11. The droplet according to claim 9, wherein the biological material is in a form of cell.

12. The droplet according to claim 11, wherein each droplet comprises one cell, and each droplet comprises at least one vector.

13. The droplet according to claim 9, wherein the droplet identification molecule further comprises a captured sequence, the captured sequence being connected to the droplet index sequence.

14. The droplet according to claim 9, further comprising a cell lysis reagent.

15. The droplet according to claim 9, wherein the droplet identification molecule is in a form of a long-chain molecule, the long-chain molecule comprising a plurality of droplet identification molecules in tandem, and
wherein the plurality of droplet index sequences on the same long-chain molecule has an identical nucleic acid sequence.

16. The droplet according to claim 15, wherein the long-chain molecule further comprises an endonuclease recognition sequence located between two adjacent droplet identification molecules of the plurality of droplet identification molecules, and
wherein the droplet further comprises an endonuclease configured to cleave the endonuclease recognition sequence.

17. The droplet according to claim 16, wherein the endonuclease recognition sequence is a double-stranded sequence, and the droplet identification molecule is a single-stranded sequence.

18. The droplet according to claim 9, wherein the droplet identification molecule is in a form of a second vector, the second vector carrying a plurality of droplet identification molecules.

19. The droplet according to claim 18, wherein the plurality of droplet identification molecules is connected to the second vector through a covalent bond or any other connection manner, and wherein the plurality of droplet identification molecules each further comprises a captured sequence, the captured sequence being connected to the droplet index sequence.

20. The droplet according to claim 19, wherein the covalent bond is a disulfide bond.

21. The droplet according to claim 19, further comprising:
a cleavage reagent capable of cleaving a connection between the plurality of droplet identification molecules and the second vector.

22. The droplet according to claim 21, wherein the cleavage reagent is DTT.

23. The droplet according to claim 9, having a water-in-oil structure.

24. A suspension, comprising a plurality of droplets according to any one of claims 9 to 23, wherein the droplet index sequences of at least two droplets of the plurality of droplets have different nucleic acid sequences.

25. A cell dispersion, comprising:
a cell; and
a long-chain molecule comprising a plurality of droplet index sequences in tandem,
wherein the plurality of droplet index sequences on the same long-chain molecule has an identical nucleic acid sequence.

26. The cell dispersion according to claim 25, wherein the cell is present in a form of single cell.

27. The cell dispersion according to claim 25, comprising a plurality of long-chain molecules, wherein the plurality of droplet index sequences on at least two long-chain molecules of the plurality of long chain molecules has different nucleic acid sequences.

28. The cell dispersion according to claim 25, wherein the long-chain molecule further comprises an endonuclease recognition sequence located between two adjacent droplet index sequences of the plurality of droplet index sequences.

29. The cell dispersion according to claim 28, wherein the endonuclease recognition sequence is a double-stranded sequence, and wherein each of the plurality of droplet index sequences is a single-stranded sequence.

30. The cell dispersion according to claim 25, wherein the long-chain molecule is in a form of DNA nanoball.

31. A cell dispersion, comprising:
a cell; and
a second vector carrying a plurality of droplet identification molecules, each of the plurality of droplet identification molecules carrying a droplet index sequence.

32. The cell dispersion according to claim 31, wherein the plurality of droplet identification molecules is connected to the second vector through a covalent bond or any other connection manner, and wherein the cell is in a form of single cell.

33. The cell dispersion according to claim 31, wherein each of the plurality of droplet identification molecules further comprises a captured sequence, the captured sequence being connected to the droplet index sequence.

34. The cell dispersion according to claim 32, wherein the covalent bond is a disulfide bond.

35. A sequencing method, comprising:
sequencing the sequencing library according to any one of claims 1 to 8 to obtain a sequencing result composed of a plurality of sequencing reads; and
classifying sources of the plurality of sequencing reads based on at least one of the cell index sequence, the droplet index sequence, or the Unique Molecular Identifier.

36. A cyclic nucleic acid molecule, comprising:
a replication-initiating sequence;
a captured sequence; and
a droplet index sequence.

37. The cyclic nucleic acid molecule according to claim 36, further comprising an endonuclease recognition sequence.

38. A method for preparing a long-chain molecule, comprising:
performing a rolling circle replication amplification on the cyclic nucleic acid molecule according to claim 36 or 37 to obtain the long-chain molecule.

39. A linear nucleic acid molecule, comprising:
a 5'-end sequence and a 3'-end sequence;
a replication-initiating sequence;
a captured sequence; and
a droplet index sequence,
wherein the 5'-end sequence and the 3'-end sequence constitute an endonuclease recognition sequence.

40. A method for analyzing single-cell nucleic acid, comprising:
providing a single-cell suspension containing dispersed single cells, and mixing the single-cell suspension with droplet identification molecules to obtain a cell suspension, each of the droplet identification molecules carrying a droplet index sequence;
placing the cell suspension, a first vector, and an oil at different positions of a microfluidic chip in such a manner that the cell suspension, the first vectors, and the oil pass through a same channel to obtain the droplet according to any one of claims 9 to 23;
performing demulsification and library construction on the droplet to obtain a sequencing library; and
sequencing and analyzing the sequencing library to obtain nucleic acid information of the single cell.

41. The method according to claim 40, wherein a concentration of the single-cell suspension ranges from 100 to 200 cells per microliter, and wherein a concentration of the droplet identification molecules ranges from 10⁵ to 10⁸ copies per microliter.

42. The method according to claim 40, wherein a concentration of the first vectors ranges from 2,000 to 3,000 per microliter.
